# EUROPEAN PATENT SPECIFICATION

(11) **EP 1 500 398 B1**
(45) Date of publication and mention of the grant of the patent: **15.11.2006**
(21) Application number: 04254356.1
(22) Date of filing: 21.07.2004
(51) Int. Cl.: A61K 36/896, A61K 36/81, A61K 36/48, A61K 36/74, A61K 33/36, A61P 35/02

(54) **Herbo-mineral formulation for refractory leukemias and lymphomas**
Kräutermineralzusammensetzung für refraktäre Leukämien und Lymphome
Composition à base d'herbes et de minéraux pour le traitement des leucémies et lymphomes résistantes

(30) Priority: 21.07.2003 GB 0317020
(43) Date of publication of application: 26.01.2005
(73) Proprietor: Sahajanand Biotech Private Limited, Surat 395 003, Gujarat (IN)
(72) Inventor: Managoli, Nandkishore, c/o Sahajanand Biotech, Saiyedpura Surat 395003 Gujarat (IN)
(74) Representative: Gaunt, Robert John

(56) References cited:
- WO-A-99/24029
- WO-A-03/006034
- GB-A- 2 388 539
- US-A1- 2003 099 725
- DATABASE BIOSIS [Online] BIOSCIENCES INFORMATION SERVICE, PHILADELPHIA, PA, US; 1990, AGRAWAL S ET AL: "PRELIMINARY OBSERVATIONS ON LEUKEMIA SPECIFIC AGGLUTININS FROM SEEDS" XP002296285 Database accession no. PREV199089129813 & INDIAN JOURNAL OF MEDICAL RESEARCH SECTION B, vol. 92, no. FEB, 1990, pages 38-42, ISSN: 0970-9568

## Description

This invention relates to a new herbo-mineral formulation which has been found to be effective for the treatment of cancer. More particularly, the formulation can be used to treat refractory leukemias, lymphomas, myelodysplastic syndrome and aplastic anemias.

The conventional treatment of cancer comprises surgery, chemotherapy and/or radiotherapy. The drugs given during chemotherapy are of necessity very powerful and, in consequence, can have serious and undesirable side-effects.
There is therefore a need for improved pharmaceutical or medicinal preparations for use in the treatment of cancer. It is the object of this invention to provide such a product.

According to this invention there is provided a pharmaceutical or medicinal preparation which comprises a mixture of a mineral and the following five herbs:
- Mineral :: Arsenic trioxide (As₂O₃)
- Herbs :: Aloe Vera (Aloe Barbedensis)
Withania Somnifera
Glycine Max
Rubia Cordifolia
Acacia Catechu
or a mixture of the mineral with the active ingredients that have been extracted from these herbs. This product has been found by the inventor to be particularly effective for the treatment of all refractory leukemias, lymphomas, myelodysplastic syndrome and aplastic anemias. The synergistic effects of the herbo-mineral preparation also stimulates the bone-marrow to produce its normal cellular constituents in leukemias, aplastic anemias and myelodysplastic syndromes, thereby causing complete remission. The preparation is preferably formulated for administration to patients as a capsule.

The ingredients for a typical herbo-mineral formulation according to this invention are set out in Table 1. It should be appreciated that the proportions of the individual herbs may be varied and the figures quoted in Table 1 are by way of illustration only. In particular, the proportions of one or more of the components may be varied in order to optimize the pharmacological effects produced by the formulation to suit the specific needs of patients being treated.

It is an important feature of the product of the present invention that it contains a mixture of a mineral with herbs / extracts from herbs, rather than being based on a single herb. A synergistic effect has been noticed between various ingredients. The synergistic activity is surprising and unexpected. Arsenic trioxide (A_{S2}O₃) is an inorganic trivalent arsenical. Pre-clinical studies have demonstrated a dose-dependent induction of apoptosis and partial differentiation in myeloid leukemia cell lines and induction of apoptosis and cell cycle arrest in lymphoid neoplasms. The activities of similar herbs are combined to optimize and enhance the pharmacological effects without increasing the adverse toxic reactions (which becomes a distinct possibility if the herbs are used singly in a concentration of 100%). The advantage of a multi-drug regimen also lies in the fact that the possibility of development of drug resistance is minimized.

Preliminary clinical trials of the product of this invention have produced definite clinical evidence of improvement in the condition of patients suffering from refractory leukemias, lymphomas, myelodysplastic syndrome and aplastic anemias. These improvements include:
i) reduction in the number of leukaemic blast cells in the peripheral blood cells as well as the bone marrow, with complete remission in refractory leukemias;
ii) normalization of the cellular components of the bone marrow in myelodysplastic syndromes due to the adaptogenic effects;
iii) increase in the number of normal cellular elements of the bone marrow in cases of aplastic anemias;
iv) reduction in the size of lymphoid neoplasms; and
v) improvement in the relevant biochemical parameters;
and more subjectively:
i) sense of well being,
ii) improvement in the psychological status of the patient,
iii) improvement in appetite,
iv) increased weight,
v) improved vigour and enthusiasm in daily activities,
vi) reduction of anorexia and cachexia, and
vii) improvement in "quality of life".

The formulation of this invention is itself effective for the treatment of cancer. It may also be used as an adjuvant to conventional modes of anticancer therapy, namely radiotherapy and/or chemotherapy. The formulation may be presented as a dietary supplement for patients diagnosed as having any type of cancer. It may also be used to create a sense of general well being and to increase the vitality in patients diagnosed as having any type of cancer; to increase the appetite, restore health and increase the lifespan of patients diagnosed as having any type of cancer, to improve the ambulatory capacity in patients diagnosed as having any type of cancer; to activate the nervous system, prevent degenerative changes, stimulate regeneration and improve the psychological status in patients diagnosed as having any type of cancer; and to stimulate metabolism, accelerate anabolism, promote catabolism thereby flushing the body of toxic metabolites and reducing the side effects of chemotherapy and radiotherapy.

### Method of extraction

Each of the herbal components of the formulation were de-seeded (wherever required), ground finely to powder form and then submitted individually to conventional solvent extraction methods.

Arsenic trioxide (As₂O₃) was purchased in pure form (99% pure) for usage in the formulation.
- Withania Somnifera and Rubia Cordifolia were extracted using methanol (root extracts)
- Acacia Catechu was extracted using saline (seed extracts).
- Aloe Vera (Aloe Emodin) was prepared by using standard extraction procedures.
- Glycine Max (Isoflavones) was prepared by using standard extraction procedures.

### Example

### A case report of a patient of chronic myeloid leukemia

| | |
|---|---|
| Age | :- 30 |
| Sex | :- Male |
| Diagnosis | :- Chronic myeloid leukemia |
| Treatment | :- Herbal medicine according to the present invention (referred to as Azuron) |
| Dosage | :- 2 capsules (450 mg per capsule) three times a day |

The patient was first seen on 27 June 2003, when he was examined, a Haemogram report was prepared and therapy started.

At that time the patient was suffering from breathlessness on exertion, easy fatigue and weakness.

| **Haemogram (27106/03)** | | |
|---|---|---|
| | **Patient Value** | **Reference Range** |
| Total W.B.C. Count | 54,500 | 5000 to 10,000 Cells/Cu mm |
| Platelet Count | 4,54,000 | 1,50,000 to 4,00,000 /Cu mm |
| Hemoglobin | 9.2 | M. 13.0 to 16.5 g/dL |

The patient's latest report was prepared on 16 June 2004. On this date there was no breathlessness, weakness or fatigue. He can work also in farm or house.

| **Haemogram (16/06/04)** | | |
|---|---|---|
| | **Patient Value** | **Reference Range** |
| Total W.B.C. Count | 5000 | 5000 to 10,000 Cells/Cu mm |
| Platelet Count | 1.63 | 1,50,000 to 4,00,000 /Cu mm |
| Hemoglobin | 10.82 | M. 13.0 to 16.5 g/dL |

The patient's improvement is attributed to the administration of Azuron. The Azuron product is a preparation according to the present invention and has the following composition:-

**AZURON**

| *Ingredient* | *Proportion (by weight)* |
|---|---|
| Arsenic Trioxide | 1mg |
| Aloe Vera | 20% |
| Withania Somnifera | 20% |
| Glycine Max | 20% |
| Rubia Cordifolia | 20% |
| Acacia Catechu | 20% |

| | |
|---|---|
| Note that arsenic trioxide, being a mineral, is used in a fixed concentration of 1 mg/capsule (in a 450 mg capsule). Its concentration is therefore not presented as a percentage value in the above list of ingredients. | |

The herbal ingredients used in the above mentioned formulation are standardized hydro-alcoholic extracts strategically combined as per the percentages mentioned, and then they are encapsulated.

### Acute Oral Toxicity Test

An acute oral toxicity test (in rats) has been carried out for capsules of the Azuron formulation. A single oral administration of Azuron was given to Sprague Dawley rats to assess its acute toxicity. In the sighting studies, Azuron did not cause mortality or signs of toxicity in females treated at 300 mg or 200 mg/kg body weight. During the main study, also at 200 mg/kg, it did not cause any mortality or signs of toxicity.

Azuron did not adversely affect the body weight gain of treated rats during the 14 day observation period, post-treatment. It also did not induce any gross pathological alternations in any of the rats, as evident at necropsy.

**Table 1 HERBO-MINERAL FORMULATION FOR REFRACTORY LEUKEMIAS AND LYMPHOMAS**

| ***Description of Ingredients*** | | | | | | |
|---|---|---|---|---|---|---|
| Sr. No. | Latin Binomial | Common Names | Distribution | Parts used | Quantity | Adverse Reactions |
| 1 | Arsenic Trioxide (As₂O₃) | Arsenic | - | - | 1 mg. per Capsule | None for the dosage used. |
| 2 | Aloe Vera (Aloe Barbedensis) | Indian Aloe, Kumari | More in the drier parts of India | Leaf juice, elio | 17 - 23% preferably 20% | None for the dosage used. |
| 3. | Glycine Max | Soya Bean | Cultivated throughout India | Seeds | 17 - 23% preferably 20% | None for the dosage used. |
| 4. | Withania Somnifera | Ashwagandha | All parts of India | Roots, leaves | 17 - 23% preferably 20% | None for the dosage used. |
| 5 | Rubia Cordifolia | Manjistha | Throughout India. | Roots | 17 - 23% preferably 20% | None for the dosage used. |
| 6. | Acacia Catechu | Kattha | Cultivated throughout India | bark | 17 - 23% preferably 20% | None for the dosage used. |

## Claims

1. A pharmaceutical or medicinal preparation comprising a mixture of arsenic trioxide (As₂O₃) and the herbs Aloe Vera (Aloe Barbedensis), Withania Somnifera, Glycine Max, Rubia Cordifolia and Acacia Catechu, or a mixture of the active ingredients that have been extracted from those herbs.

2. A pharmaceutical or medicinal preparation as claimed in claim 1, for use in the treatment of cancer.

3. A pharmaceutical or medicinal preparation as claimed in claim 1, for use in the treatment of refractory leukemias.

4. A pharmaceutical or medicinal preparation as claimed in claim 1, for use in the treatment of lymphomas.

5. A pharmaceutical or medicinal preparation as claimed in claim 1, for use in the treatment of myelodysplastic syndrome.

6. A pharmaceutical or medicinal preparation as claimed in claim 1, for use in the treatment of aplastic anemias.

7. A pharmaceutical or medicinal preparation as claimed in claim 1, for use as an adjuvant to conventional modes of anticancer therapy, namely radiotherapy and/or chemotherapy.

8. A pharmaceutical or medicinal preparation as claimed in claim 1, for use in improving the quality of life of patients suffering from cancer.

9. A pharmaceutical or medicinal preparation as claimed in any one of claims 1 to 8, wherein the amount of the herbs is as follows:
| | |
|---|---|
| Arsenic Trioxide (As₂O₃) | 1 mg. per Capsule |
| Aloe Vera (Aloe Barbedensis) | 17 - 23% preferably 20% |
| Glycine Max | 17 - 23% preferably 20% |
| Withania Somnifera | 17 - 23% preferably 20% |
| Rubia Cordifolia | 17 - 23% preferably 20% |
| Acacia Catechu | 17-23% preferably 20% |

10. A pharmaceutical or medicinal preparation as claimed in any one of claims 1 to 8, wherein the amount of the herbs is as follows:
| Arsenic Trioxide (As₂O₃) | 1 mg. per Capsule |
|---|---|
| Aloe Vera (Aloe Barbedensis) | 20% |
| Glycine Max | 20% |
| Withania Somnifera | 20% |
| Rubia Cordifolia | 20% |
| Acacia Catechu | 20% |

11. A dietary supplement for patients diagnosed as having any type of cancer, which includes a pharmaceutical or medicinal preparation as claimed in claim 1.

## Patentansprüche

1. Pharmazeutisches oder medizinisches Präparat, umfassend ein Gemisch aus Arsentrioxid (As₂O₃) und den Kräuterpflanzen Aloe vera (Aloe barbedensis), Withania somnifera, Glycine max, Rubia cordifolia und Acacia catechu, oder ein Gemisch aus Wirkstoffen, die aus diesen Kräuterpflanzen extrahiert worden sind.

2. Pharmazeutisches oder medizinisches Präparat nach Anspruch 1 zur Verwendung bei der Behandlung von Krebs.

3. Pharmazeutisches oder medizinisches Präparat nach Anspruch 1 zur Verwendung bei der Behandlung von refraktären Leukämien.

4. Pharmazeutisches oder medizinisches Präparat nach Anspruch 1 zur Verwendung bei der Behandlung von Lymphomen.

5. Pharmazeutisches oder medizinisches Präparat nach Anspruch 1 zur Verwendung bei der Behandlung von myelodysplastischem Syndrom.

6. Pharmazeutisches oder medizinisches Präparat nach Anspruch 1 zur Verwendung bei der Behandlung von aplastischen Anämien.

7. Pharmazeutisches oder medizinisches Präparat nach Anspruch 1 zur Verwendung als ein Hilfsmittel für konventionelle Arten der Antikrebstherapie, nämlich Strahlentherapie und/oder Chemotherapie.

8. Pharmazeutisches oder medizinisches Präparat nach Anspruch 1 zur Verwendung bei der Verbesserung der Lebensqualität von Patienten, die an Krebs leiden.

9. Pharmazeutisches oder medizinisches Präparat nach einem der Ansprüche 1 bis 8, wobei die Menge der Kräuterpflanzen folgendermaßen ist:
| Arsentrioxid (As₂O₃) | 1 mg pro Kapsel |
|---|---|
| Aloe vera (Aloe barbedensis) | 17 - 23 %, bevorzugt 20 % |
| Glycine max | 17 - 23 %, bevorzugt 20 % |
| Withania somnifera | 17 - 23 %, bevorzugt 20 % |
| Rubia cordifolia | 17 - 23 %, bevorzugt 20 % |
| Acacia catechu | 17 - 23 %, bevorzugt 20 % |

10. Pharmazeutisches oder medizinisches Präparat nach einem der Ansprüche 1 bis 8, wobei die Menge der Kräuterpflanzen folgendermaßen ist:
| Arsentrioxid (As₂O₃) | 1 mg pro Kapsel |
|---|---|
| Aloe vera (Aloe barbedensis) | 20 % |
| Glycine max | 20 % |
| Withaniasomnifera | 20 % |
| Rubia cordifolia | 20 % |
| Acacia catechu | 20 % |

11. Nahrungsergänzungsmittel für Patienten mit der Diagnose irgendeiner Krebsart, das ein pharmazeutisches oder medizinisches Präparat nach Anspruch 1 umfaßt.

## Revendications

1. Préparation pharmaceutique ou médicale comprenant un mélange de trioxyde d'arsenic (As₂O₃) et les herbes Aloe Vera (Aloe Barbedensis), Withania Somnifera, Glycine Max, Rubia Cordifolia et Acacia Catechu, ou un mélange des substances actives qui ont été extraites de ces herbes.

2. Préparation pharmaceutique ou médicale selon la revendication 1 pour utilisation dans le traitement du cancer.

3. Préparation pharmaceutique ou médicale selon la revendication 1 pour utilisation dans le traitement des leucémies réfractaires.

4. Préparation pharmaceutique ou médicale selon la revendication 1 pour utilisation dans le traitement des lymphomes.

5. Préparation pharmaceutique ou médicale selon la revendication 1 pour utilisation dans le traitement du syndrome myélodysplasique.

6. Préparation pharmaceutique ou médicale selon la revendication 1 pour utilisation dans le traitement des anémies aplasiques.

7. Préparation pharmaceutique ou médicale selon la revendication 1 pour utilisation comme adjuvant aux modes conventionnels de thérapie anticancéreuse, c'est-à-dire la radiothérapie et/ou la chimiothérapie.

8. Préparation pharmaceutique ou médicale selon la revendication 1 pour utilisation en vue d'améliorer la qualité de vie de patients atteints du cancer.

9. Préparation pharmaceutique ou médicale selon l'une quelconque des revendications 1 à 8, dans laquelle la quantité d'herbes est la suivante :
| Trioxyde d'arsenic (As₂O₃) | 1 mg par capsule |
|---|---|
| Aloe Vera (Aloe Barbedensis) | 17 à 23%, de préférence 20% |
| Glycine Max | 17 à 23%, de préférence 20% |
| Withania Somnifera | 17 à 23%, de préférence 20% |
| Rubia Cordifolia | 17 à 23%, de préférence 20% |
| Acacia Catechu | 17 à 23%, de préférence 20% |

10. Préparation pharmaceutique ou médicale selon l'une quelconque des revendications 1 à 8, dans laquelle la quantité d'herbes est la suivante :
| Trioxyde d'arsenic (As₂O₃) | 1 mg par capsule |
|---|---|
| Aloe Vera (Aloe Barbedensis) | 20% |
| Glycine Max | 20% |
| Withania Somnifera | 20% |
| Rubia Cordifolia | 20% |
| Acacia Catechu | 20% |

11. Complément alimentaire pour patients diagnostiqués comme ayant n'importe quel type de cancer, qui comprend une préparation pharmaceutique ou médicale selon la revendication 1.
